# EUROPEAN PATENT SPECIFICATION

(11) **EP 1 781 210 B1**
(45) Date of publication and mention of the grant of the patent: **16.01.2013**
(21) Application number: 05789032.9
(22) Date of filing: 11.07.2005
(51) Int. Cl.: A61F 2/06

(54) **COMPOSITE VASCULAR GRAFT INCLUDING BIOACTIVE AGENT COATING AND BIODEGRADABLE SHEATH**
VERBUNDGEFÄSSIMPLANTAT MIT BIOAKTIVER WIRKSTOFFBESCHICHTUNG UND BIOLOGISCH ABBAUBARER HÜLLE
GREFFE VASCULAIRE COMPOSITE COMPRENANT UN REVETEMENT D'AGENT BIOACTIF ET UNE GAINE BIODEGRADABLE

(30) Priority: 12.07.2004 US 889432
(43) Date of publication of application: 09.05.2007
(73) Proprietor: Boston Scientific Limited, Barbados, West Indies (BB)
(72) Inventor: MI LYN TAN, Sharon, Allston, MA 02134 (US)
(74) Representative: Intès, Didier Gérard André
(86) International application number: PCT/US2005/024418
(87) International publication number: WO 2006/017204

(56) References cited:
- WO-A-95/29647
- WO-A-02/056790
- US-A1- 2003 060 871
- US-A1- 2004 236 415
- US-B1- 6 379 382

## Description

### CROSS-REFERENCE TO RELATED APPLICATIONS

This International Application claims the benefit of U.S. Application No. 10/889,432, filed July 12, 2004.

### FIELD OF THE INVENTION

The present invention relates to implantable medical devices which inhibit or reduce bacterial growth during their use in a living body. More particularly, the present invention relates to composite vascular grafts which incorporate bioactive agents to deliver therapeutic materials and/or to inhibit or reduce bacterial growth during and following the introduction of the graft to the implantation site in the body.

### BACKGROUND OF THE INVENTION

In order to repair or replace diseased or damaged blood vessels it is well known to use implantable vascular grafts in the medical arts. These vascular grafts, which are typically polymeric tubular structures, may be implanted during a surgical procedure or maybe interluminally implanted in a percutaneous procedure.

Such medical procedures employing vascular grafts introduce a foreign object into a patient's vascular system. Therefore, the risk of infection must be addressed in any such procedure.

Vascular graft infection is reported to occur in from about 1% to 6% of the procedures. More significantly, vascular graft infections are associated with a high mortality rate of between 25% to 75%. Moreover, morbidity rates for vascular graft infections are in the range of between 40% and 75%. Infections caused by vascular grafts are also known to prolong hospital stays, thereby greatly increasing the cost of medical care.

Numerous factors contribute to the risk of vascular graft infection. Such factors include the degree of experience of the surgeon and operating room staff. The age of the patent and the degree to which the patient is immunocompromised also are strong risk factors with respect to vascular graft insertion. Other common factors associated with vascular graft infection risks include sterility of the skin of the patient, as well as the materials being implanted.

It has been found that the mechanism of infection for many implanted devices is attributed to local bacterial contamination during surgery. Bacteria on the device produce an extracellular slime matrix/biofilm during colonization, which coats the polymer surface. This biofilm protects the bacteria against the patient's defense mechanisms. The biofilm layer also reduces the penetration of antibiotics.

The most common infectious agents are: *staphylococcus aureus, pseudomonas aeruginosa,* and *staphylococcus epidermis.* These agents have been identified in over 75% of all reported vascular infections. Both *staphylococcus aureus* and *pseudomonas aeruginosa,* show high virulence and can lead to clinical signs of infection early in the post-operative period (less than four months). It is this virulence that leads to septicemia and is one main factor in the high mortality rates. *Staphylococcus epidermis* is described as a low virulence type of bacterium. It is late occurring, which-means it can present clinical signs of infection up to five years post-operative. This type of bacterium has been shown to be responsible for up to 60% of all vascular graft infections. Infections of this type often require total graft excision, debridement of surrounding tissue, and revascularization through an uninfected route.

Such high virulence organisms are usually introduced at the time of implantation. For example, some of the *staphylococcus* strains (including *staphylococcus aureus*) have receptors for tissue ligands such as fibrinogen molecules which are among the first deposits seen after implantation of a graft. This tissue ligand binding provides a way for the bacteria to be shielded from the host immune defenses as well as systemic antibiotics. The bacteria can then produce polymers in the form of a polysaccharide that can lead to the aforementioned slime layer on the outer surface of the graft. In this protective environment, bacterial reproduction occurs and colonies form within the biofilm that can shed cells to surrounding tissues (Calligaro, K. and Veith, Frank, Surgery, 1991 V110-No. 5, 805-811). Infection can also originate from transected lymphatics, from inter-arterial thrombus, or be present within the arterial wall.

There are severe complications as a result of vascular graft infections. For example, anastonomic disruption due to proteolytic enzymes that the more virulent organisms produce can lead to a degeneration of the arterial wall adjacent to the anastomosis. This can lead to a pseudoaneurism which can rupture and cause hemodynamic instability. A further complication of a vascular graft infection can be distal styptic embolisms, which can lead to the loss of a limb, or aortoenteric fistulas, which are the result of a leakage from a graft that is infected and that leads to gastrointestinal bleeding (Greisler, H., Infected Vascular Grafts. Maywood, IL, 33-36).

Desirably, it would be beneficial to prevent any bacteria from adhering to the graft, or to the immediate area surrounding the graft at the time of implantation. It would further be desirable to prevent the initial bacterial biofilm formation described above by encouraging normal tissue ingrowth within the tunnel, and by protecting the implant itself from the biofilm formation.

It is known to incorporate antimicrobial agents into a medical device. For example, prior art discloses an ePTFE vascular graft, a substantial proportion of the interstices of which contain a coating composition that includes: a biomedical polyurethane; poly(lactic acid), which is a biodegradable polymer; and the antimicrobial agents, chlorhexidine acetate and pipracil. The prior art further describes an ePTFE hernia patch which is impregnated with a composition including silver sulfadiazine and chlorhexidine acetate and poly(lactic acid).

Moreover, prior art is known, which discloses a stent or vascular prosthesis having an overlying biodegradable coating layer that contains a drug. The coating layer is disclosed as including an anticoagulant drug, and, optionally, other additives such as an antibiotic substance.

Further prior art describes a medical implant wherein an antimicrobial agent penetrates the exposed surfaces of the implant and is impregnated throughout the material of the implant. The medical implant may be a vascular graft and the material of the implant may be polytetrafluoroethylene (PTFE). The antimicrobial agent is selected from antibiotics, antiseptics and disinfectants.

Moreover, there is prior art that discloses that silver, which is a known antiseptic agent, can be deposited onto the surface of a porous polymeric substrate via silver ion assisted beam deposition prior to filling the pores of a porous polymeric material with an insoluble, biocompatible, biodegradable material. This prior art further discloses that antimicrobials can be integrated into the pores of the polymeric substrate. The substrate may be a porous vascular graft of ePTFE.

It is also known to provide an anti-infective medical article including a hydrophilic polymer having silver chloride bulk distributed therein. The hydrophilic polymer may be a laminate over a base polymer. Preferred hydrophilic polymers are disclosed as melt processible polyurethanes. The medical article may be a vascular graft. A disadvantage of this graft is that it is not formed of ePTFE, which is known to have natural antithrombogenic properties. A further disadvantage is that the hydrophilic polyurethane matrix into which the silver salt is distributed does not itself control the release of silver into the surrounding body fluid and tissue at the implantation site of the graft.

Furthermore, there is prior art describing an implantable medical device that can include a stent structure, a layer of bioactive material posited on one surface of the stent structure, and a porous polymeric layer for controlled release of a bioactive material which is posited over the bioactive material layer. The thickness of the porous polymeric layer is described as providing this controlled release. The medical device can further include another polymeric coating layer between the stent structure and the bioactive material layer. This polymeric coating layer is disclosed as preferably being formed of the same polymer as the porous polymeric layer. Silver can be included as the stent base metal or as a coating on the stent base metal. Alternatively, silver can be in the bioactive layer or can be posited on or impregnated in the surface matrix of the porous polymeric layer. Polymers of polytetrafluoroethylene and bioabsorbable polymers can be used. A disadvantage of this device is that it is not designed to achieve fast tissue ingrowth within the tunnel to discourage initial bacterial biofilm formation.

Further prior art describes an antimicrobial vascular graft made with a porous antimicrobial fabric formed by fibers which are laid transverse to each other, and which define pores between the fibers. The fibers may be of ePTFE. Ceramic particles are bound to the fabric material, the particles including antimicrobial metal cations thereon, which may be silver ions. The ceramic particles are exteriorly exposed and may be bound to the graft by a polymeric coating material, which may be a biodegradable polymer. A disadvantage of this device is that the biodegradable coating layer does not provide sufficient rigidity during implantation for an outer graft layer.

Another known example of vascular graft is disclosed in PCT patent application n° WO 02/056790 A2.

There is a need for additional antimicrobial vascular grafts. In particular, there is a need for multi-layered vascular grafts which incorporate antimicrobial agents and, optionally, other therapeutic or diagnostic agents that can be controllably released upon implantation from biodegradable materials in the graft to suppress infection and to prevent biofilm formation. It would also be desirable to provide such grafts with sufficient rigidity in the tissue-contacting outer layer and with good cellular communication between the blood and the perigraft tissue in the luminal layer.

### SUMMARY OF THE INVENTION

The present invention provides a composite vascular graft having a bioactive agent incorporated therein. The graft includes a flexible, porous tubular graft member that may be an ePTFE tube and/or a textile. The porous tubular graft member may be covered with one or more biodegradable, bioactive agent coating layers. Desirably, the bioactive agent coating layer includes an antimicrobial agent. The graft further includes a biodegradable sheath disposed over the one or more bioactive agent coating layers. The sheath has a rigidity greater than the flexible tubular graft member; and is biodegradable to expose the bioactive agent coating layer so as to re-establish the flexibility of the tubular graft member. The sheath optionally includes a bioactive agent, such as an antimicrobial agent.

The present invention also provides a method for forming a composite vascular graft which incorporates bioactive agents therein. The method can include the steps of providing a porous, flexible tubular graft member; and applying a biodegradable coating material having at least one bioactive agent incorporated therein to the graft member so as to form one or more overlying biodegradable, bioactive agent coating layers. A biodegradable sheath, which optionally includes a bioactive agent, is then disposed over the one or more bioactive agent coating layers overlying the graft member.

### BRIEF DESCRIPTION OF THE DRAWINGS

Figure 1A is a schematic longitudinal cross-sectional representation of an embodiment of the vascular graft of the present invention, wherein the graft includes a single bioactive agent coating layer.
Figure 1B is a schematic longitudinal cross-sectional representation of a further embodiment of the vascular graft of the present invention wherein the graft includes multiple bioactive agent coating layers.
Figure 2 is a schematic longitudinal cross-sectional representation of yet another embodiment of the vascular graft of the present invention, wherein the biodegradable sheath of the composite graft includes bioactive agents therewithin.
Figure 3 is a perspective view of a tubular vascular graft according to the present invention.
Figure 4 is a cross-sectional showing of an embodiment of a stent/graft composite of the present invention wherein the inner porous tubular graft member is an ePTFE tube.
Figure 5 is a perspective view of a textile tubular graft member useful in the composite graft of the present invention.
Figure 6 is a schematic showing of a conventional weave pattern useful for the textile tubular graft member in Figure 5.
Figure 7 is a perspective showing of a biodegradable sheath in tubular configuration useful in the composite graft of the present invention.
Figure 8 is a perspective showing of a biodegradable sheath in sheet-like configuration useful in the composite graft of the present invention.

### DETAILED DESCRIPTION OF THE INVENTION

In preferred embodiments of the present invention, the implantable composite device is a multi-layered tubular structure, which is particularly suited for use as a vascular graft. The prosthesis preferably includes at least one porous, flexible tubular graft member made of a textile and/or ePTFE. Furthermore, the prosthesis preferably includes one or more biodegradable coating layers disposed over the graft member and designed to regulate delivery of an antimicrobial agent associated therewith to the site of implantation. The prosthesis also includes a biodegradable sheath disposed over the one or more coating layers overlying the graft member.

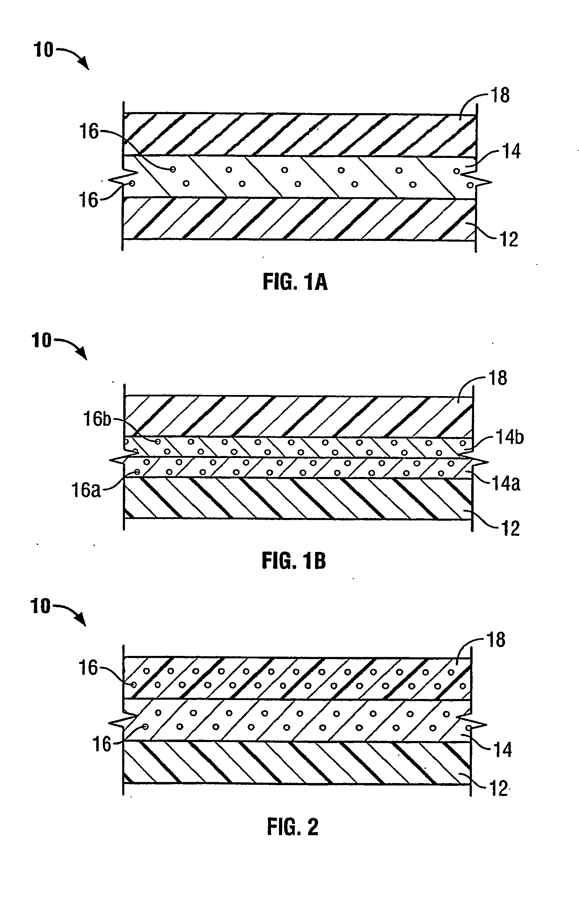

Figure 1A shows vascular graft 10 of the present invention. As noted above, the present invention takes the preferred embodiment of a tubular graft having a composite structure. The layers shown in Figure 1 represent the tubular members forming the composite structure. However, it may be appreciated that the present invention also contemplates other implantable multi-layer prosthetic structures such as vascular patches, blood filters, film wraps for implantable devices such as stents, hernia repair fabrics and plugs and other such devices where such structures may be employed. As shown in Figure 1A, the composite device 10 of the present invention includes a tubular flexible vascular graft member 12, which is porous and made of a textile and/or ePTFE. A biodegradable, bioactive agent coating layer 14 covers the graft member 12. Biodegradable coating layer 14 permits controlled delivery of bioactive agents 16 associated with coating layer 14 therethrough. These bioactive agents 16 are preferably distributed substantially evenly throughout the bulk of the bioactive agent coating layer 14, as will be described in greater detail below. Bioactive agents 16 desirably include antimicrobial agents. Device 10 of the present invention further includes a biodegradable sheath 18, which has a rigidity greater than that of flexible graft member 12. After implantation, sheath 18 biodegrades upon exposure to blood and/or other physiological fluids. This biodegradation of the sheath 18 decreases the rigidity of the graft so as to re-establish the flexibility of the tubular graft member 12. Once the sheath has degraded, it exposes bioactive agent coating layer 14. Desirably, antimicrobial agents are posited on or incorporated within coating layer 14 to reduce infection after implantation. Sheath 18 may be in a tubular configuration and placed over the graft member 12 or may be in a sheet-like configuration and wrapped about the tubular graft member 12, as further described below. The biodegradable sheath 18 is desirably flexible and slightly elastic in nature to allow it to be placed on top of or wrapped about the vascular graft 12.

With reference now to Figure 1B, in one aspect of the present invention the bioactive agent coating is applied to graft member 12 in multiple coating layers, such as 14a and 14b. It is well within the contemplation of the present invention that coating layers 14a and 14b may contain the same or different bioactive agents 16. For example, as shown in the embodiment in Fig. 1B, bioactive agent 16a in coating layer 14a is an antibiotic agent, whereas bioactive agent 16b in coating layer 14b is an antiseptic agent. It can be appreciated that these multiple coating layers can be applied onto graft member 12 for a longer term anti-infective effect. Bioactive agent coating layer 14a is exposed after bioactive agent coating layer 14b has been biodegraded. Desirably, the bioactive agent coating layers are both biodegradable, as well as bioresorbable.

Referring now to Figure 2, in another aspect of the present invention, biodegradable sheath 18 also includes one or more bioactive agents. In desired embodiments, the bioactive agents in the biodegradable sheath include at least one antimicrobial agent such that antimicrobial agents are controllably released from the biodegradable sheath immediately upon implantation to reduce infection after implantation. Once the sheath biodegrades and is desirably resorbed, the one or more bioactive agent coating layers 14 are exposed for a longer term anti-infective effect.

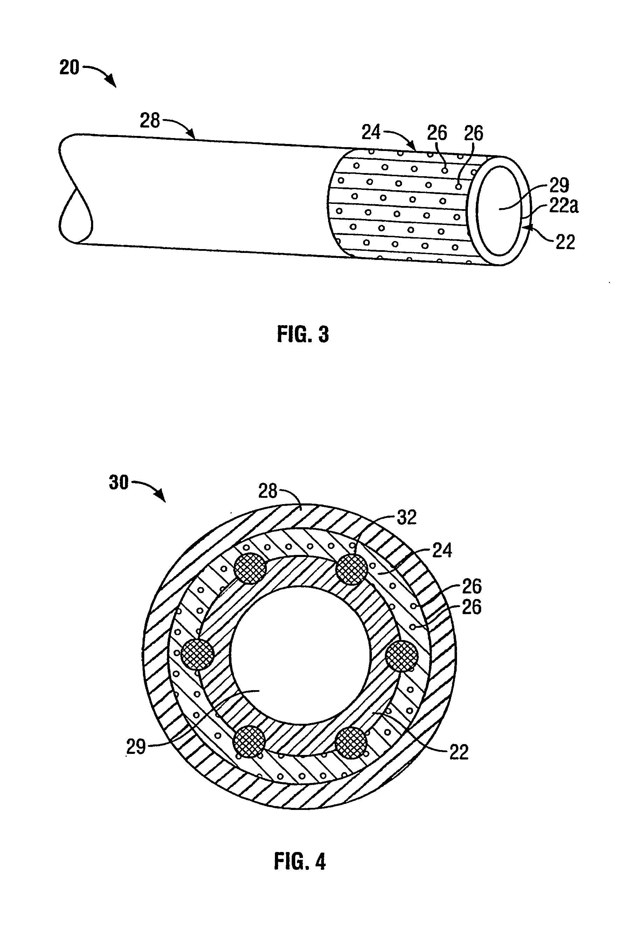

Referring now to Figure 3, a preferred embodiment of a composite tubular graft of the present invention is shown, wherein the layers shown in Figure 1A represent the tubular members in Figure 3 forming the composite structure. Device 20 includes an inner porous tubular graft member 22, which is flexible; and a medial coating layer 24 disposed coaxially thereover. Medial layer 24 includes bioactive agent 26 which is preferably distributed substantially evenly throughout the bulk of the biodegradable matrix of layer 24. An outer tubular biodegradable sheath member 28 is disposed coaxially over biodegradable bioactive coating layer 24. As will be described in further detail below, the porous flexible tubular graft member 22 can be an ePTFE tube and/or a textile. A central lumen 29 extends throughout the tubular composite graft 20 defined further by the inner wall 22a of luminal tube 22, which permits the passage of blood through graft 20 once the graft is properly implanted in the vascular system.

It is well within the contemplation of the present invention that a stent can be interposed between the tubular members of the graft of the present invention. With reference to Figure 4, a stent/graft composite device 30 of the present invention is shown. Device 30 includes inner porous tubular graft member 22, which in the present figure is depicted as an ePTFE tubular member. Device 30 also includes at least one medial, biodegradable, bioactive agent coating layer 24 disposed coaxially over graft member 22. As described above, coating layer 24 includes at least one bioactive agent which can be controllably released from the biodegradable matrix of coating layer 24. Composite device 30 further includes a biodegradable tubular sheath member 28 which is disposed coaxially over tubular member 24. As described above and as shown in Figure 2, sheath member 28 can also include bioactive agents. In desired embodiments, the bioactive agents associated with coating layer 24 and optionally with biodegradable sheath 28, include an antimicrobial agent that can be controllably released from coating layer 24 and sheath 28 depending on the rate of hydrolysis of the bonds within these biodegradable members. Central lumen 29 extends throughout tubular composite graft 30. An expandable stent 32 may be interposed between inner ePTFE tubular member 22 and biodegradable coating layer 24. Stent 32, which may be associated with the graft of the present invention, is used for increased support of the blood vessel and increased blood flow through the area of implantation. It is noted that increased radial tensile strength at the outer sheath member 28 enables the graft to support, for example, radial expansion of stent 32, when present. In order to facilitate hemodialysis treatment, a significant number of patients suffering from hypertension or poor glycemic control in diabetes will have a synthetic vascular graft surgically implanted between the venous and arterial systems. Typically, these grafts become occluded over time. In these instances, a covered stent across the venous anastomotic site in patients with significant stenosis may aid in prolonging the patency of these grafts, which would avoid painful and typically expensive surgical revisions. For these reasons, it is well within the contemplation of the present invention that a stent covered with or incorporated within the vascular graft of the present invention may be useful for AV access.

The bioactive agents may include antimicrobial agents. In one embodiment, the antimicrobial agents are antibiotic or antiseptic agents, or combinations thereof The antibiotic agents can be of the type including, but not limited to, ciprofloxacin, vancomycin, minocycline, rifampin and other like agents, as well as combinations thereof.

Suitable antiseptic agents include, but are not limited to, the following: silver agents, chlorhexidine, triclosan, iodine, benzalkonium chloride and other like agents, as well as combinations thereof.

For example, silver is an antiseptic agent that has been shown *in vitro* to inhibit bacterial growth in several ways. For example, it is known that silver can interrupt bacterial growth by interfering with bacterial replication through a binding of the microbial DNA, and also through the process of causing a denaturing and inactivation of crucial microbial metabolic enzymes by binding to the sulfhydryl groups (Tweten, K., J. of Heart Valve Disease 1997, V6, No. 5, 554-561). It is also known that silver causes a disruption of the cell membranes of blood platelets. This increased blood platelet disruption leads to increased surface coverage of the implants with platelet cytoskeletal remains. This process has been shown to lead to an encouragement of the formation of a more structured (mature state) pannus around the implant. This would likely discourage the adhesion and formation of the biofilm produced by infectious bacteria due to a faster tissue ingrowth time (Goodman, S. et al, 24th Annual Meeting of the society for Biomaterials, April 1998, San Diego, CA; pg. 207).

The silver agent can be a silver metal ion such as silver iodate, silver iodide, silver nitrate, and silver oxide. These silver ions are believed to exert their effects by disrupting respiration and electron transport systems upon absorption into bacterial or fungal cells. Antimicrobial silver ions are useful for *in vivo* use because they are not substantially absorbed into the body, and typically pose no hazard to the body.

Referring again to Figure 1A, the aforementioned antiseptic or antibiotic bioactive agents 16 can be used alone or in combination of two or more of them. These agents 16 can be posited on coating layer 14 or can be dispersed throughout coating layer 14. The amount of each antimicrobial or antibiotic bioactive agent 16 used to posit onto or to impregnate the coating layer 14 varies to some extent, but is at least of an effective concentration to inhibit the growth of bacterial and fungal organisms.

As noted above, in one aspect of the present invention, composite device 10 includes an ePTFE graft member as the porous graft member 12 depicted in Figure 1A. PTFE exhibits superior biocompatibility and low thrombogenicity, which makes it particularly useful as vascular graft material. Desirably, the ePTFE graft member is a tubular structure 22, as depicted in Figure 4. The ePTFE material has a fibrous state, which is defined by interspaced nodes interconnected by elongated fibrils. The space between the node surfaces that is spanned by the fibrils is defined as the internodal distance. In the present invention, the internodal distance in a luminal ePTFE graft member is desirably about 70 to about 90 microns in order to achieve fast tissue ingrowth within the tunnel to discourage initial bacterial biofilm formation. When the term "expanded" is used to describe PTFE, i.e. ePTFE, it is intended to describe PTFE which has been stretched, in accordance with techniques which increase the internodal distance and, concomitantly, porosity. The stretching may be done uni-axially, bi-axially, or multi-axially. The nodes are stretched apart by the stretched fibrils in the direction of the expansion. Methods of making conventional longitudinally expanded ePTFE are well known in the art.

It is further contemplated that the ePTFE may be a physically modified ePTFE tubular structure having enhanced axial elongation and radial expansion properties of up to 600% by linear dimension. The physically modified ePTFE tubular structure is able to be elongated or expanded and then returned to its original state without an elastic force existing therewithin. Additional details of physically-modified ePTFE and methods for making the same can be found in commonly assigned Application Title "ePTFE Graft With Axial Elongation Properties", assigned U.S. Application No. 09/898,418, filed on July 3, 2001, published on January 9, 2003 as U.S. Application Publication No. 2003-0009210A1.

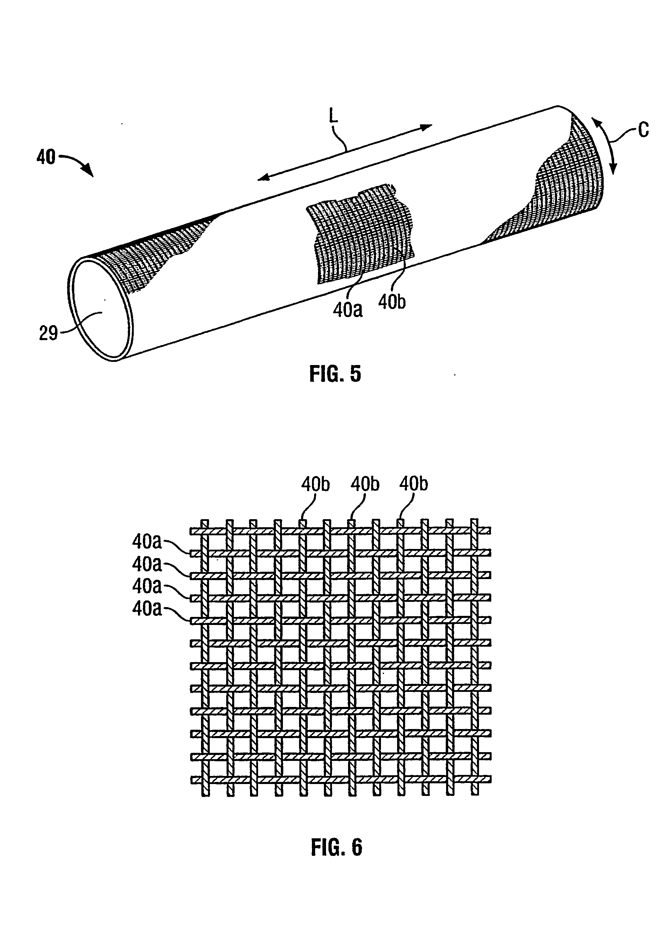

As noted above, in another aspect of the present invention, composite device 10 includes a textile graft member as the porous graft member 12 in Figure 1A. As will be described in further detail below, virtually any textile construction can be used for the graft 12, including weaves, knits, braids, filament windings, spun fibers and the like. Any weave pattern in the art, including, simple weaves, basket weaves, twill weaves, velour weaves and the like may be used. With reference to Figures 5 and 6, the weave pattern of a textile tubular graft member 40 shown in Figure 5 includes warp yarns 40a running along the longitudinal length (L) of the graft and fill yarns 40b running around the circumference (C) of the graft, the fill yarns being at approximately 90 degrees to one another with fabrics flowing from the machine in the warp direction. A central lumen 29 extends throughout the tubular graft member 40, which permits the passage of blood through the composite vascular graft of the present invention once it is assembled and is properly implanted in the vascular system.

Any type of textile products can be used as yarns for a textile graft member. Of particular usefulness in forming a textile graft member for the composite device of the present invention are synthetic materials such as synthetic polymers. Synthetic yarns suitable for use in the textile graft member include, but are not limited to, polyesters, including PET polyesters, polypropylenes, polyethylenes, polyurethanes and polytetrafluoroethylenes. The yarns may be of the mono-filament, multi-filament, spun-type or combinations thereof. The yarns may also be flat, twisted or textured, and may have high, low or moderate shrinkage properties or combinations thereof. Additionally, the yarn type and yarn denier can be selected to meet specific properties desired for the prosthesis, such as porosity and flexibility. The yarn denier represents the linear density of the yarn (number of grams mass divided by 9,000 meters of length). Thus, a yarn with a small denier would correspond to a very fine yarn, whereas a yarn with a large denier, e.g., 1,000, would correspond to a heavy yarn. The yarns used for the textile graft member of the device of the present invention may have a denier from about 20 to about 200, preferably from about 30 to about 100. Desirably, the yarns are polyester, such as polyethylene terephthalate (PET). Polyester is capable of shrinking during a heat-set process, which allows it to be heat-set on a mandrel to form a generally circular shape.

After forming the textile layer of the present invention, it is optionally cleaned or scoured in a basic solution of warm water. The textile is then rinsed to remove any remaining detergent, and is then compacted or shrunk to reduce and control in part the porosity of the textile layer. Porosity of a textile material is measured on the Wesolowski scale and by the procedure of Wesolowski. In this test, a textile test piece is clamped flatwise and subjected to a pressure head of about 120 mm of mercury. Readings are obtained which express the number of mm of water permeating per minute through each square centimeter of fabric. A zero reading represents absolute water impermeability and a value of about 20,000 represents approximate free flow of fluid.

The porosity of the textile layer is often about 5,000 to about 17,000 on the Wesolowski scale. The textile layer may be compacted or shrunk in the wale direction to obtain the desired porosity. A solution of organic component, such as hexafluoroisopropanol or trichloroacetic acid, and a halogenated aliphatic hydrocarbon, such as methylene chloride, can be used to compact the textile graft by immersing it into the solution for up to 30 minutes at temperatures from about 15°C to about 160°C.

Yarns of the textile layer may be one ply or multi-ply yarns. Multi-ply yarns may be desirable to impart certain properties onto the drawn yarn, such as higher tensile strengths for the textile layer.

A further aspect of the composite device of the present invention relates to the biodegradable, bioactive agent coating layer shown as layer 14 in Figure 1A. In one embodiment, the bioactive agent coating is applied to the porous tubular graft member as one or more coating layers. For example, a coating material can be applied (prior to polymerization) as a liquid to the outside surface of an ePTFE and/or textile graft member by such means as dipping, spraying or painting.

The coating layer may be comprised of natural, modified natural or synthetic polymers, copolymers, block polymers, as well as combinations thereof. It is noted that a polymer is generally named based on the monomer it is synthesized from. Examples of suitable biodegradable polymers or polymer classes include fibrin, collagen, elastin, celluloses, gelatin, vitronectin, fibronectin, laminin, reconstituted basement membrane matrices, starches, dextrans, alginates, hyaluronic acid, poly(lactic acid), poly(glycolic acid), polypeptides, glycosaminoglycans, their derivatives and mixtures thereof. For both glycolic acid and lactic acid, an intermediate cyclic dimer is typically prepared and purified, prior to polymerization. These intermediate dimers are called glycolide and lactide, respectively.

Other useful biodegradable polymers or polymer classes for the bioactive agent coating layer include the following: polydioxanones, polyoxalates, poly(α-esters), polyanhydrides, polyacetates, polycaprolactones, poly(orthoesters), polyamino acids, polyamides and mixtures and copolymers thereof.

Additional useful biodegradable polymers for the bioactive agent coating layer include, stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates and mixtures thereof. Binary and ternary systems are contemplated.

Factors affecting the mechanical performance of *in vivo* biodegradable polymers are well known to the polymer scientist, and include monomer selection, initial process conditions, and the presence of additives. Biodegradation has been accomplished by synthesizing polymers that have unstable linkages in the backbone, or linkages that can be safely oxidized or hydrolyzed in the body. The most common chemical functional groups having this characteristic are ethers, esters, anhydrides, orthoesters and amides.

As described above, the biodegradable coating layer includes a bioactive agent. In one desired embodiment, the bioactive agent is an antimicrobial agent. For example, the antimicrobial agent can be an antibiotic or antiseptic agent. Examples of suitable antibiotic and antiseptic agents for use in the present invention are provided above.

The bioactive agent is desirably evenly distributed throughout the bulk of the biodegradable coating layer and is controllably released from the biodegradable coating layer to the site of implantation of the graft by hydrolysis of chemical bonds in the biodegradable polymer. It is also contemplated that a bioactive agent can be posited on the coating layer.

A solution of biodegradable material that includes a monomer (or an intermediate cyclic dimer) on which the biodegradable polymer is based can be applied as a coating to the external side of the ePTFE and/or textile graft member. This can be accomplished by such means as dipping, spraying, painting, etc. A bioactive agent can be blended into the wet or fluid biodegradable material to form a coating mixture which is then applied to the porous tubular graft member by a spraying process, for example. Alternatively, the bioactive agent may be applied in powdered form to wet or fluid biodegradable material after the biodegradable material has been applied as a coat to the porous tubular graft member, but prior to its polymerization.

In preparing the biodegradable, bioactive agent coating layer, a solution or fluid of a biocompatible, biodegradable material can be formed. For example, extracellular matrix proteins which are used in fluid/solution may be soluble. However, some materials may be difficult to dissolve in water. Collagen, for example, is considered insoluble in water, as is gelatin at ambient temperature. To overcome such difficulties, collagen or gelatin may preferably formed at an acidic pH, i.e. at a pH less than 7 and, preferably, at a pH of about 2 to about 4. The temperature range at which such fluid/solutions are formed is between about 4°C to about 40°C, and preferably about 30°C - 35°C.

In situations where the bioactive agent is insoluble in the wet or fluid biodegradable coating material, the agent may be finely subdivided as by grinding with a mortar and pestle. The finely subdivided bioactive agent can then be distributed desirably substantially evenly throughout the bulk of the wet or fluid biodegradable coating material before cross-linking or cure solidifies the coating layer.

It is well within the contemplation of the present invention that the coating layer can be combined with various carrier, drug, prognostic, or therapeutic materials. For example, the coating layer can be combined with any of the following therapeutic agents: antimicrobial agents, such as the antibiotic agents and antiseptic agents listed above; anti-thrombogenic agents, such as heparin, heparin derivatives, urokinase, and PPack (dextrophenylalanine proline, arginine, chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents, such as dexamethasone, prednisolone, corticosterone, budesonide, estrogen, sulfasalazine, and mesalamine); antineoplastics/anti-proliferative/anti-miotic agents (such as paclitaxel, 5-flurouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick anti-platelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bi-functional molecules consisting of a growth factor and a cytotoxin, bi-functional molecules consisting of an antibody and a cytotoxin); cholesterol-lowering agents; vasodilating agents; and agents which interfere with andogenous or vascoactive mechanisms. In addition, cells which are able to survive within the body and are dispersed within the coating layer may be therapeutically useful. These cells themselves may be therapeutically useful or they may be selected or engineered to produce and release therapeutically useful compositions.

In other embodiments, bioactive agents associated with the composite device of the present invention may be genetic agents. Examples of genetic agents include DNA, anti-sense DNA, and anti-sense RNA. DNA encoding one of the following may be particularly useful in association with an implantable device according to the present invention: (a) tRNA or RRNA to replace defective or deficient endogenous molecules; (b) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor; (c) cell cycle inhibitors; (d) thymidine kinase and other agents useful for interfering with cell proliferation; and (e) the family of bone morphogenic proteins. Moreover, DNA encoding molecules capable of inducing an upstream or downstream effect of a bone morphogenic protein may be useful.

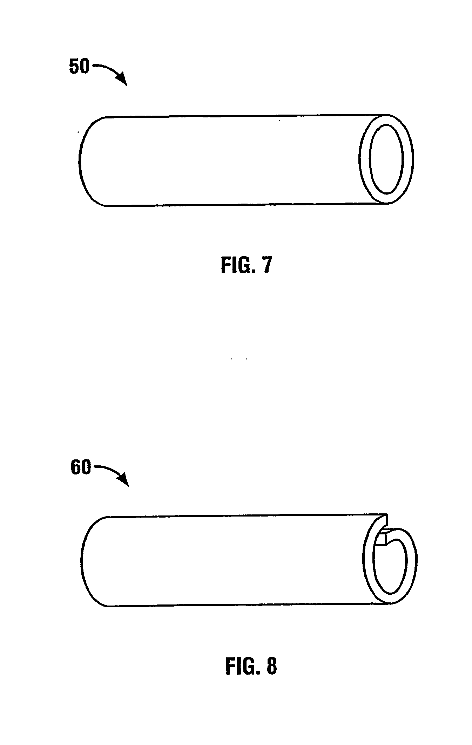

A further aspect of the present invention relates to the biodegradable sheath shown as layer 18 in Figure 1A. In one embodiment, the biodegradable sheath is comprised of a material selected from, but not limited to, the following: polylactides, polyanhydrides, polyvinyl alcohol, polyvinylpyrolidone, polyglycols, gelatin derivatives and combinations thereof. The biodegradable sheath can have a tubular or sheet-like configuration for disposal over the bioactive coating layer. For example, referring to Figure 7 of the present invention, there is shown a biodegradable sheath in a tube-like configuration 50 used in combination with a tubular composite vascular graft of the present invention. Specifically, the tube 50 can be placed over the bioactive coating layer overlying the porous, flexible tubular graft member.

Alternatively, the biodegradable sheath can be in a sheet-like configuration as shown in Figure 8. Sheath 60 shown in Figure 8 is used in combination with a tubular composite vascular graft of the present invention. Specifically, the sheath 60 can be wrapped about the bioactive coating layer overlying the porous, flexible tubular graft member. The sheet 60 is seamed along the longitudinal axis.

The sheath provides a desired degree of initial rigidity to the flexible tubular textile and/or ePTFE graft member during implantation. After implantation, the sheath biodegrades upon exposure to blood and/or other physiological fluids. The biodegradation of the sheath decreases the rigidity of the graft and re-establishes the flexibility of the graft member. After the sheath has degraded, it exposes the underlying bioactive agent coating layer which is desirably incorporated with antimicrobial agents to reduce infection after implantation. In embodiments where multiple bioactive agent coating layers are present, each coating layer controllably releases bioactive agents associated therewith after the coating layer overlying it is resorbed. This provides a longer term anti-infective effect.

The biodegradable sheath of the composite graft of the present invention can include bioactive agents. For example, the biodegradable sheath can be incorporated with antimicrobial agents so as to controllably release the antimicrobial agents immediately upon implantation.

In one of the embodiments of the present invention, it is contemplated that a dry, finely subdivided antimicrobial agent may be blended with wet or fluid material to form a mixture which is used to impregnate the pores of a porous biodegradable sheath. Alternatively, it is contemplated that air pressure or other suitable means may then be employed to disperse the antimicrobial agent substantially evenly within the filled pores.

In one example, a bioactive agent or drug can be incorporated into the sheath in the following manner: mixing into a fluid material used to make the sheath, a crystalline, particulate material like salt or sugar that is not soluble in a solvent used to form the sheath; casting the solution with particulate material into a film or sheet; and then applying a second solvent, such as water, to dissolve and remove the particulate material, thereby leaving a porous sheath. The sheath may then be placed into a solution containing a bioactive agent in order to fill the pores. Preferably, a vacuum would be pulled on the sheath to insure that the bioactive agent applied to it is received into the pores.

It is also contemplated that the bioactive agent or drug may be encapsulated in microparticles, such as microspheres, microfibers or microfibrils, which can then be incorporated into or on the sheath. Various methods are known for encapsulating bioactive agents or drugs within microparticles or microfibers (see Patrick B. Deasy, Microencapsulation and Related Drug Processes, Marcel Dekker, Inc., New York, 1984). In one example, a suitable microsphere for incorporation can have a diameter of about 10 microns or less. The microsphere could be contained within the biodegradable polymeric matrix of the sheath. The microparticles containing the bioactive agent can be incorporated within the sheath by adhesively positioning them onto the sheath material or by mixing the microparticles with a fluid or gel and flowing them into the sheath layer. The fluid or gel mixed with the microparticles could, for example, be a carrier agent designed to improve the cellular uptake of the bioactive agent incorporated into the sheath. Moreover, it is well within the contemplation of the present invention that carrier agents, which can include hyaluronic acid, may be incorporated within each of the embodiments of the present invention so as to enhance cellular uptake of the bioactive agent(s) associated with the device.

The microparticles may have a polymeric wall surrounding the bioactive agent or a matrix containing the bioactive agent and optional carrier agents, which due to the potential for varying thicknesses of the polymeric wall and for varying porosities and permeabilities suitable for containing a bioactive agent, there is provided the potential for an additional mechanism for controlling the release of a therapeutic agent.

Moreover, microfibers or microfibrils, which may be loaded with the bioactive agent by extrusion, can be adhesively layered or woven into the sheath material for drug delivery.

The bioactive agents, which can optionally be associated with the biodegradable sheath of the composite graft of the present invention, may be selected from drugs, prognostic agents, carrier agents, therapeutic agents, and genetic agents. Suitable bioactive agents include, but are not limited to, growth factors, anti-coagulant substances, stenosis inhibitors, thrombo-resistant agents, antibiotic agents, anti-tumor agents, anti-proliferative agents, growth hormones, antiviral agents, anti-angiogenic agents, angiogenic agents, anti-mitotic agents, anti-inflammatory agents, cell cycle regulating agents, genetic agents, cholesterol-lowering agents, vasodilating agents, agents that interfere with endogenous vasoactive mechanisms, hormones, their homologs, derivatives, fragments, pharmaceutical salts and combinations thereof. Specific examples of such agents are provided above.

As described above, a further aspect of the present invention relates to a method of making the inventive composite vascular graft. The method includes the steps of providing a flexible, porous tubular graft member, such as an ePTFE and/or textile graft member; and applying a biodegradable coating material to the porous tubular graft member so as to form one or more overlying coating layers, wherein the biodegradable coating material has at least one bioactive agent incorporated therein. The method further includes disposing a biodegradable sheath over the one or more coating layers overlying the ePTFE and/or textile graft member.

Generally, tubular textile layers are manufactured in a single long tube and cut to a pre-determined length. To cut the textile layer, a laser would be desirably used, which cuts and fuses the ends simultaneously. The textile layer is typically cleaned, desirably with sodium dodecyl sulfate and then rinsed with deionized water. The textile layer can then be placed over a cylindrical mandrel and heat set to precisely set the diameter and to remove any creases or wrinkles. Typically, heat setting is carried out at the temperature range from about 125°C to about 225°C using a convection oven for a time of 20 minutes. Any known means for heating may be used.

Alternatively, the composite device of the present invention may be formed by expanding a thin wall PTFE inner luminal tube at a relatively high degree of elongation, on the order of approximately between 400% and 2,000% elongation and preferably from about between 700% and 900%. The inner luminal tube is desirably expanded over a cylindrical mandrel, such as a stainless steel mandrel at a temperature of between room temperature and 337°C (640°F), preferably about 260°C (500°F). The luminal tube is preferably, but not necessarily fully sintered after expansion. Sintering is typically accomplished at a temperature of between 337°C (640°F) and 426°C (800°F), preferably at about 348°C (660°F) and for a time of between about 5 minutes to 30 minutes, preferably about 15 minutes. The resulting luminal tube formed by this method desirably exhibits an IND of greater than 40 microns, and in particular between 40 and 100 microns, most desirably between 70 to about 90 microns, spanned by a moderate number of fibrils. Such a microporous structure is sufficiently large so as to promote enhanced cell endothelization once blood flow is established through the graft. Such cell endothelization enhances the long-term patency of the graft.

The combination of the luminal ePTFE and/or textile tube over the mandrel is then employed as a substrate over which the biodegradable, bioactive coating layer can be disposed. In particular, the biodegradable, bioactive coating layer can be applied as a fluid coating material on the external surface of the luminal tube by such means as dipping, spraying or painting. The bioactive agent coating can be applied in a single layer or in multiple layers. Within the bioactive agent coating material is preferably substantially evenly dispersed a bioactive agent, which may be in dry powdered form.

The biodegradable sheath, which can be in the form of a tube or sheet, is then disposed over the bioactive agent coating layer(s). For example, the tube or sheet may correspond to a porous, biodegradable polymeric matrix, wherein the pores can optionally be filled with a bioactive agent. The interior diameter of a biodegradable tubular sheath member is selected so that it may be easily, but tightly disposed over the outside diameter of the coated graft member. In one embodiment, the sheath is cross-linked and bonds to the underlying bioactive agent coating layer. It is further contemplated that the biodegradable sheath can be secured to the coated graft member using techniques that would avoid degrading or damaging the bioactive agents in the coating layer(s). For example, where silver metal ions are the bioactive agents, it may be suitable to sinter the composite structure formed between the coated, tubular graft member and the tubular sheath using similar parameters to those described above.

Alternatively, the biodegradable sheath may be securably affixed to the coated graft member by means of a bonding agent. The bonding agent may include various biocompatible, elastomeric bonding agents such as urethanes, styrene/isobutylene/styrene block copolymers (SIBS), silicones, and combinations thereof. Once the composite prosthesis is formed, one or more layers of elastic tubing, preferably silicone, can then be placed over this composite structure. This holds the composite structure together and assures that complete contact and adequate pressure is maintained for bonding purposes.

While the invention has been described in relation to the preferred embodiments with several examples, it will be understood by those skilled in the art that various changes may be made within the scope of the invention as defined in the appended claims.

## Claims

1. A composite vascular graft comprising:
a porous, flexible tubular graft member (12);
a biodegradable, bioactive agent coating layer (14) disposed over said graft member (12); said coating layer (14) being provided with at least one bioactive agent (16) incorporated therein;
the composite vascular graft being **characterized in that** it comprises:
a biodegradable sheath (18) disposed over said coating layer (14), said sheath (18) having a rigidity greater than said flexible tubular graft member (12); and being biodegradable to expose said coating layer (14) so as to re-establish the flexibility of said tubular graft member (12).

2. The vascular graft of claims 1, wherein said bioactive agent (16) is an antimicrobial agent.

3. The vascular graft of claim 2, wherein said antimicrobial agent is an antibiotic or antiseptic agent.

4. The vascular graft of claim 3, wherein said antibiotic agent is selected from the group consisting of ciprofloxacin, vancomycin, minocycline, rifampin and combinations thereof.

5. The vascular graft of claim 3, wherein the antiseptic agent is selected from the group consisting of a silver agent, chlorhexidine, triclosan, iodine, benzalkonium chloride, and combinations thereof.

6. The vascular graft of any one of claims I to 5, wherein said porous tubular graft member (12) comprises ePTFE material.

7. The vascular graft of any one of claims 1 to 6, wherein said porous tubular graft member (12) comprises a textile material.

8. The vascular graft of claim 7, wherein said textile material comprises a construction selected from the group consisting of weaves, braids, filament windings, spun fibers and combinations thereof.

9. The vascular graft of claim 7, wherein said textile material is formed from synthetic yarns (40a, 40b) selected from the group consisting of polyesters, PET polyesters, polypropylenes, polyethylenes, polyurethanes, polytetrafluoroethylenes and combinations thereof.

10. The vascular graft of any one of claims I to 9, wherein the biodegradable, bioactive agent coating layer (14) is comprised of a natural, modified natural or synthetic polymer.

11. The vascular graft of claim 10, wherein said polymer is selected from the group consisting of fibrin, collagen, celluloses, gelatin, vitronectin, fibronectin, laminin, reconstituted basement membrane matrices, starches, dextrans, alginates, hyaluronic acid, poly(lactic acid), poly(glycolic acid), polypeptides, glycosaminoglycans, their derivatives and mixtures thereof.

12. The vascular graft of claim 10, wherein said polymer is selected from the group consisting of polydioxanoes, polyoxalates, poly(α-esters), polyanhydrides, polyacetates, polycaprolactones, poly(orthoesters), polyamino acids, polyamides and mixtures and copolymers thereof.

13. The vascular graft of claim 10, wherein said polymer is selected from the group consisting of stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkunoates and mixtures thereof.

14. The vascular graft of any one of claims 1 to 13, wherein said bioactive agent coating (14) is applied to said tubular graft member (12).

15. The vascular graft of any one of claims 1 to 14, wherein said bioactive agent coating is applied in multiple layers (14a, 14b).

16. The vascular graft of any one of claims I to 15, wherein the biodegradable sheath (18) has a tubular or sheet-like configuration for disposal over said bioactive agent coating layer (14).

17. The vascular graft of any one of claims 1 to 16, wherein the biodegradable sheath (18) is comprised of a material selected from the group consisting of polylactides, polyanhydrides, polyvinyl alcohol, polyvinylpyrolidone, polyglycols, gelatin derivatives, and combinations thereof.

18. The vascular graft of any one of claims 1 to 17, wherein the biodegradable sheath (18) includes at least one antimicrobial agent (16).

19. A method of making a vascular graft according to claim 1 for delivery of an antimicrobial agent associated therewith to a site of implantation of said graft, said method comprising the steps of:
providing a porous, flexible tubular graft member (12);
applying a biodegradable coating material to said porous tubular graft member (12) so as to form one or more overlying biodegradable, bioactive agent coating layers (14, 14a, 14b), said biodegradable coating material having at least one bioactive agent (16) incorporated therein; and
disposing a biodegradable sheath (18) over said one or more overlying coating layers(14, 14a, 14b).

20. The method of claim 19, wherein the disposing step includes providing the sheath (18) in a tubular configuration and placing said sheath (18) over the one or more coating layers (14, 14a, 14b) overlying said graft member (12).

21. The method of claim 19, wherein the disposing step includes providing the sheath (18) in a sheet-like configuration and wrapping the sheet over the one or more coating layers (14, 14a, 14b) overlying said graft member (12).

22. The method of any one of claims 19 to 21. further comprising the step of interposing a prosthetic stent (32) between said tubular graft member (12) and said one or more bioactive agent coating layers (14, 14a, 14b).

23. The method of any one of claims 19 to 22, further comprising the step of incorporating said bioactive agent (16) into said biodegradable coating material.

24. The method of any one of claims 19 to 23, wherein said bioactive agent (16) is an antimicrobial agent selected from the group consisting of antiseptic agents, antibiotic agents, and combinations thereof.

## Patentansprüche

1. Verbundgefäßprothese, umfassend:
ein poröses, flexibles röhrenförmiges Prothesenelement (12),
eine biologisch abbaubare Beschichtungsschicht (14) aus bioaktivem Wirkstoff, die auf dem Prothesenelement (12) angeordnet ist, wobei die Beschichtungsschicht (14) mit wenigstens einem darin eingelagerten bioaktiven Wirkstoff (16) versehen ist, wobei die Verbundgefäßprothese **dadurch gekennzeichnet ist, daß** sie umfaßt:
eine biologisch abbaubare Hülse (18), die über der Beschichtungsschicht (14) angeordnet ist, wobei die Hülse (18) eine größere Steifigkeit aufweist als das flexible röhrenförmige Prothesenelement (12) und biologisch abbaubar ist, um die Beschichtungsschicht (14) freizulegen, um die Flexibilität des röhrenförmigen Prothesenelementes (12) wiederherzustellen.

2. Gefäßprothese nach Anspruch 1, wobei der bioaktive Wirkstoff (16) ein antimikrobieller Wirkstoff ist.

3. Gefäßprothese nach Anspruch 2, wobei der antimikrobielle Wirkstoff ein antibiotischer oder antiseptischer Wirkstoff ist.

4. Gefäßprothese nach Anspruch 3, wobei der antibiotische Wirkstoff aus der aus Ciprofloxacin, Vancomycin, Minocyclin, Rifampin und Kombinationen davon bestehenden Gruppe ausgewählt ist.

5. Gefäßprothese nach Anspruch 3, wobei der antiseptische Wirkstoff aus der aus Silberwirkstoff, Chlorhexidin, Triclosan, Jod, Benzalkoniumchlorid und Kombinationen davon bestehenden Gruppe ausgewählt ist.

6. Gefäßprothese nach einem der Ansprüche 1 bis 5, wobei das poröse röhrenförmige Prothesenelement (12) ePTFE-Material umfaßt.

7. Gefäßprothese nach einem der Ansprüche 1 bis 6, wobei das poröse röhrenförmige Prothesenelement (12) ein textiles Material umfaßt.

8. Gefäßprothese nach Anspruch 7, wobei das textile Material eine Konstruktion umfaßt, die aus der aus Geweben, Borten, Filamentwindungen, Spinnfasern und Kombinationen davon bestehenden Gruppe ausgewählt ist.

9. Gefäßprothese nach Anspruch 7, wobei das textile Material aus synthetischen Garnen (40a, 40b) gebildet ist, die aus der aus Polyestern, PET-Polyestern, Polypropylenen, Polyethylenen, Polyurethanen, Polytetrafluorethylenen und Kombinationen davon bestehenden Gruppe ausgewählt ist.

10. Gefäßprothese nach einem der Ansprüche 1 bis 9, wobei die biodegradierbare Beschichtungsschicht (14) aus bioaktivem Wirkstoff ein natürliches, modifiziertes natürliches oder synthetisches Polymer umfaßt.

11. Gefäßprothese nach Anspruch 10, wobei das Polymer aus der aus Fibrin, Collagen, Zellulosen, Gelatin, Vitronectin, Fibronectin, Laminin, wiederhergestellte Basalmembranmatrizes, Stärken, Dextranen, Alginaten, Hyaluronsäure, Poly(milchsäure), Poly(glycolsäure), Polypeptiden, Glycosaminglycanen, deren Derivaten und Mischungen davon bestehenden Gruppe ausgewählt ist.

12. Gefäßprothese nach Anspruch 10, wobei das Polymer aus der aus Polydioxanen, Polyoxalaten, Poly(α-Estern), Polyanhydriden, Polyacetaten, Polycaprolactonen, Poly(orthoestern), Polyaminosäuren, Polyamiden und Mischungen und Copolymeren davon bestehenden Gruppe ausgewählt ist.

13. Gefäßprothese nach Anspruch 10, wobei das Polymer aus der aus Stereopolymeren von L- und D-Milchsäure, Copolymeren von bis-(p-Carboxyphenoxy)-Propansäure und Sebacinsäure, Sebacinsäurecopolymeren, Copolymeren von Caprolacton, Poly(milchsäure)/Poly(glycolsäure)/Polyethylenglycol Copolymeren, Copolymeren von Polyurethan und Poly(milchsäure), Copolymeren von Polyurethan und Poly(milchsäure), Copolymeren von α-Aminosäuren, Copolymeren von α-Aminosäuren und Capronsäure, Copolymeren von α-Benzylglutamat und Polyethylenglycol, Copolymeren von Succinat und Poly(glycolen), Polyphosphazen, Polyhydroxyalkanoaten und Mischungen davon bestehenden Gruppe ausgewählt ist.

14. Gefäßprothese nach einem der Ansprüche 1 bis 13, wobei die Beschichtung (14) aus bioaktivem Wirkstoff auf das röhrenförmige Prothesenelement (12) aufgebracht ist.

15. Gefäßprothese nach einem der Ansprüche 1 bis 14, wobei die Beschichtung aus bioaktivem Wirkstoff in mehreren Schichten (14a, 14b) aufgebracht ist.

16. Gefäßprothese nach einem der Ansprüche 1 bis 15, wobei die biologisch abbaubare Hülse (18) eine röhrenförmige oder blattartige Konfiguration zum Ablagern über der Beschichtungsschicht (14) aus bioaktivem Wirkstoff aufweist.

17. Gefäßprothese nach einem der Ansprüche 1 bis 16, wobei die biologisch abbaubare Hülse (18) aus einem Material besteht, das aus der aus Polylactiden, Polyanhydriden, Polyvinylalkohol, Polyvinylpyrolidon, Polyglycolen, Gelatinderivaten und Kombinationen davon bestehenden Gruppe ausgewählt ist.

18. Gefäßprothese nach einem der Ansprüche 1 bis 17, wobei die biologisch abbaubare Hülse (18) wenigstens einen antimikrobiellen Wirkstoff (16) umfaßt.

19. Verfahren zum Herstellen einer Gefäßprothese nach Anspruch 1 zur Abgabe eines antimikrobiellen Wirkstoffes, der damit an einem Implantationsort der Prothese assoziiert ist, wobei das Verfahren die Schritte umfaßt:
Bereitstellen eines porösen, flexiblen röhrenförmigen Prothesenelementes (12),
Aufbringen eines biologisch abbaubaren Beschichtungsmaterials auf das poröse röhrenförmige Prothesenelement (12), um eine oder mehrere überlagernde biologisch abbaubare Beschichtungsschichten (14, 14a, 14b) aus bioaktivem Wirkstoff zu bilden, wobei das biologisch abbaubare Beschichtungsmaterial wenigstens einen darin eingelagerten bioaktiven Wirkstoff (16) aufweist, und
Anordnen einer biologisch abbaubaren Hülse (18) über der einen oder den mehreren Beschichtungsschichten (14, 14a, 14b).

20. Verfahren nach Anspruch 19, wobei der Anordnungsschritt umfaßt:
Bereitstellen der Hülse (18) in einer röhrenförmigen Konfiguration und Plazieren der Hülse (18) über der einen oder den mehreren Beschichtungsschichten (14, 14a, 14b), die das Prothesenelement (12) überlagern.

21. Verfahren nach Anspruch 19, wobei der Anordnungsschritt umfaßt:
Bereitstellen der Hülse (18) in einer blattartigen Konfiguration und Umwickeln des Blattes über die eine oder die mehreren Beschichtungsschichten (14, 14a, 14b), die das Prothesenelement (12) überlagern.

22. Verfahren nach einem der Ansprüche 19 bis 21, ferner umfassend den Schritt des Einfügens eines prothetischen Stents (32) zwischen das röhrenförmige Prothesenelement (12) und die eine oder mehreren Beschichtungsschichten (14, 14a, 14b) aus bioaktivem Wirkstoff.

23. Verfahren nach einem der Ansprüche 19 bis 22, ferner umfassend den Schritt des Einlagerns des bioaktiven Wirkstoffes (16) in das biologisch abbaubare Beschichtungsmaterial.

24. Verfahren nach einem der Ansprüche 19 bis 23, wobei der bioaktive Wirkstoff (16) ein antimikrobieller Wirkstoff ist, der aus der aus antiseptischen Wirkstoffen, antibiotischen Wirkstoffen und Kombinationen davon bestehenden Gruppe ausgewählt ist.

## Revendications

1. Greffon vasculaire composite comprenant :
un élément de greffe tubulaire, souple, poreux (12) ;
une couche de revêtement d'agent bioactif biodégradable (14) déposée sur ledit élément de greffe (12) ; ladite couche de revêtement (14) étant fournie avec au moins un agent bioactif (16) incorporé dans celle-ci ;
le greffon vasculaire composite étant **caractérisé en ce qu'**il comprend :
une gaine biodégradable (18) disposée sur ladite couche de revêtement (14), ladite gaine (18) ayant une rigidité supérieure audit élément de greffe tubulaire souple (12) ; et étant biodégradable pour exposer ladite couche de revêtement (14) de manière à rétablir la flexibilité dudit élément de greffe tubulaire (12).

2. Greffon vasculaire selon la revendication 1, dans lequel ledit agent bioactif (16) est un agent antimicrobien.

3. Greffon vasculaire selon la revendication 2, dans lequel ledit agent antimicrobien est un agent antibiotique ou antiseptique.

4. Greffon vasculaire selon la revendication 3, dans lequel ledit agent antibiotique est choisi dans le groupe constitué par la ciprofloxacine, la vancomycine, la minocycline, la rifampine et leurs combinaisons.

5. Greffon vasculaire selon la revendication 3, dans lequel l'agent antiseptique est choisi dans le groupe constitué par un agent à base d'argent, la chlorhexidine, le triclosan, l'iode, le chlorure de benzalkonium et leurs combinaisons.

6. Greffon vasculaire selon l'une quelconque des revendications 1 à 5, dans lequel ledit élément de greffe tubulaire poreux (12) comprend un matériau ePTFE.

7. Greffon vasculaire selon l'une quelconque des revendications 1 à 6, dans lequel ledit élément de greffe tubulaire poreux (12) comprend une matière textile.

8. Greffon vasculaire selon la revendication 7, dans lequel ladite matière textile comprend une construction choisie dans le groupe constitué par les tissages, les tresses, les enroulements de filament, les fibres filées et leurs combinaisons.

9. Greffon vasculaire selon la revendication 7, dans lequel ladite matière textile est formée à partir de fils synthétiques (40a, 40b) choisis dans le groupe constitué par les polyesters, les polyesters de PET, les polypropylènes, les polyéthylènes, les polyuréthanes, les polytétrafluoroéthylènes et leurs combinaisons.

10. Greffon vasculaire selon l'une quelconque des revendications 1 à 9, dans lequel la couche de revêtement d'agent bioactif, biodégradable (14) se compose d'un polymère naturel, naturel modifié ou synthétique.

11. Greffon vasculaire selon la revendication 10, dans lequel ledit polymère est choisi dans le groupe constitué par la fibrine, le collagène, les celluloses, la gélatine, la vitronectine, la fibronectine, la laminine, les matrices membranaires basales reconstituées, les amidons, les dextranes, les alginates, l'acide hyaluronique, un poly(acide lactique), un poly(acide glycolique), les polypeptides, les glycosaminoglycanes, leurs dérivés et des mélanges de ceux-ci.

12. Greffon vasculaire selon la revendication 10, dans lequel ledit polymère est choisi dans le groupe constitué par les polydioxanones, les polyoxalates, les poly(α-esters), les polyanhydrides, les polyacétates, les polycaprolactones, les poly(orthoesters), les acides polyaminés, les polyamides et des mélanges et des copolymères de ceux-ci.

13. Greffon vasculaire selon la revendication 10, dans lequel ledit polymère est choisi dans le groupe constitué par les stéréopolymères d'acide L- et D-lactique, les copolymères d'acide bis(p-carboxyphénoxy)propane et d'acide sébacique, les copolymères d'acide sébacique, les copolymères de caprolactone, les copolymères de poly(acide lactique)/poly(acide glycolique)/polyéthylène glycol, les copolymères de polyuréthane et de poly(acide lactique), les copolymères de polyuréthane et de poly(acide lactique), les copolymères d'acides α-aminés, les copolymères d'acides α-aminés et d'acide caproïque, les copolymères de glutamate d'α-benzyle et de polyéthylène glycol, les copolymères de succinate et de poly(glycols), un polyphosphazène, les polyhydroxyalcanoates, et des mélanges de ceux-ci.

14. Greffon vasculaire selon l'une quelconque des revendications 1 à 13, dans lequel ledit revêtement d'acide bioactif (14) est appliqué sur ledit élément de greffe tubulaire (12).

15. Greffon vasculaire selon l'une quelconque des revendications 1 à 14, dans lequel ledit revêtement d'agent biologique est appliqué en multiples couches (14a, 14b).

16. Greffon vasculaire selon l'une quelconque des revendications 1 à 15, dans lequel la gaine biodégradable (18) a une configuration tubulaire ou en forme de feuille pour sa mise en place sur ladite couche de revêtement d'agent bioactif (14).

17. Greffon vasculaire selon l'une quelconque des revendications 1 à 16, dans lequel la gaine biodégradable (18) se compose d'une matière choisie dans le groupe constitué par les polylactides, les polyanhydrides, un alcool polyvinylique, une polyvinylpyrrolidone, les polyglycols, les dérivés de la gélatine, et leurs combinaisons.

18. Greffon vasculaire selon l'une quelconque des revendications 1 à 17, dans lequel la gaine biodégradable (18) comprend au moins un agent antimicrobien (16).

19. Procédé de fabrication d'un greffon vasculaire selon la revendication 1, pour la délivrance d'un agent antimicrobien associé à celle-ci au niveau d'un site d'implantation de ladite greffe, ledit procédé comprenant les étapes consistant à :
utiliser un élément de greffe tubulaire, souple, poreux (12) ;
appliquer un matériau de revêtement biodégradable sur ledit élément de greffe tubulaire poreux (12) de manière à former une ou plusieurs couches de revêtement d'agent bioactif, biodégradable superposées (14, 14a, 14b), ledit matériau de revêtement biodégradable comprenant au moins un agent bioactif (16) incorporé dans celui-ci ; et
disposer une gaine biodégradable (18) sur ladite ou lesdites couches de revêtement superposées (14, 14a, 14b).

20. Procédé selon la revendication 19, dans lequel l'étape de disposition comprend la fourniture de la gaine (18) selon une configuration tubulaire et le placement de ladite gaine (18) sur une ou plusieurs couches de revêtement (14, 14a, 14b) recouvrant ledit élément de greffe (12).

21. Procédé selon la revendication 19, dans lequel l'étape de disposition comprend la fourniture de la gaine (18) selon une configuration en forme de feuille et l'enveloppement de la feuille sur une ou plusieurs couches de revêtement (14, 14a, 14b) recouvrant ledit élément de greffe (12).

22. Procédé selon l'une quelconque des revendications 19 à 21, comprenant en outre l'étape consistant à intercaler un stent prothétique (32) entre ledit élément de greffe tubulaire (12) et ladite ou lesdites couches de revêtement d'agent bioactif (14, 14a, 14b).

23. Procédé selon l'une quelconque des revendications 19 à 22, comprenant en outre l'étape consistant à incorporer ledit agent bioactif (16) dans ledit matériau de revêtement biodégradable.

24. Procédé selon l'une quelconque des revendications 19 à 23, dans lequel ledit agent bioactif (16) est un agent antimicrobien choisi dans le groupe constitué par les agents antiseptiques, les agents antibiotiques, et leurs combinaisons.
